# EUROPÄISCHE PATENTSCHRIFT

(11) **EP 4 243 681 B1**
(45) Veröffentlichungstag und Bekanntmachung des Hinweises auf die Patenterteilung: **19.08.2026**
(21) Anmeldenummer: 21806958.1
(22) Anmeldetag: 09.11.2021
(51) Int. Cl.: A61B 5/022, A61B 5/1455, A61B 5/00, A61B 5/024, A61B 5/021, A61B 5/1495

(54) **VERFAHREN UND MESSVORRICHTUNG ZUR KONTINUIERLICHEN, NICHT-INVASIVEN BESTIMMUNG ZUMINDEST EINES HERZ-KREISLAUFPARAMETERS**
METHOD AND MEASURING DEVICE FOR CONTINUOUSLY NON-INVASIVELY DETERMINING AT LEAST ONE CARDIOVASCULAR PARAMETER
PROCÉDÉ ET DISPOSITIF DE MESURE POUR LA DÉTERMINATION NON INVASIVE CONTINUE D'AU MOINS UN PARAMÈTRE CARDIOVASCULAIRE

(30) Priorität: 12.11.2020 AT 509862020
(43) Veröffentlichungstag der Anmeldung: 20.09.2023
(73) Patentinhaber: CNSystems Medizintechnik GmbH, 8020 Graz (AT)
(72) Erfinder: FORTIN, Jürgen, 8043 Graz (AT)
(74) Vertreter: Babeluk Patentanwälte GmbH
(86) Internationale Anmeldenummer: PCT/AT2021/060422
(87) Internationale Veröffentlichungsnummer: WO 2022/099339

(56) Entgegenhaltungen:
- US-A1- 2012 245 471
- US-A1- 2013 060 152
- US-A1- 2018 289 271

## Beschreibung

Die Erfindung betrifft ein Verfahren und eine Messvorrichtung zur kontinuierlichen, nicht-invasiven Bestimmung zumindest eines Herz-Kreislaufparameters, vorzugsweise des arteriellen Blutdrucks, an einer Extremität, die eine Arterie enthält, mit einem Aufnahmeelement, das an der Extremität anbringbar ist und geeignet ist die Extremität zumindest teilweise zu umfassen, sowie mit einer sich am Aufnahmeelement abstützenden, auf die Extremität wirkenden, flexiblen Blase, die mit einem Fluid gefüllt ist.

Die kontinuierliche, nicht-invasive Messung von Herz-Kreislaufparametern, insbesondere des Blutdruckes, stellt bis heute eine große Herausforderung an die Messtechnik dar. Ein Schwerpunkt der Forschung liegt seit Jahrzehnten an sogenannten "Cuffless" oder auch "Ubiquitous" Messverfahren und Geräten. Diese Verfahren vermessen unterschiedliche pulsatile Körpersignale, ohne dabei mit einer Manschette einen störenden Druck auf den Körper auszuüben. Insbesondere seit dem Durchbruch verschiedener "smarter" Sensoren wie Fitnessarmbänder, Smart-Watches aber auch intelligente Textilien oder Körperwaagen etc. will man neben der Pulsfrequenz auch andere Herz-Kreislaufparameter gewinnen.

Diese Verfahren errechnen den Blutdruck meist aus Zeitdifferenzen, die sich aus Signalen ableiten lassen, die an zumindest zwei unterschiedlichen Körperstellen abgeleitet werden. Im konkreten vermisst man die Zeit, die ein Puls braucht, um von einer distalen Körperstelle zu einer proximalen Körperstelle zu wandern. Diese Zeit in der Literatur wird als "Pulse Transit Time" oder "Pulse Arrival Time" bezeichnet.

Die US 8 100 835 B2 beschreibt eine sogenannte "Pulse Decomposition Analyse", die den Puls in eine vorwärts und in eine rückwärts gerichtete Pulswelle zerlegt. Die zeitlichen Unterschiede werden gemessen und sollen ein Maß für den Blutdruck sein. Der Vorteil dieser Methode ist, dass nur ein Sensor verwendet werden muss.

Es gibt auch Verfahren, die aus den pulsatilen Signalen eines einzelnen Sensors den Blutdruck berechnen wollen. Die US 2017 0360314 A1 beschreibt eine Methode sowie ein Gerät, wo aus der Vermessung der Pulswelle der Blutdruck kontinuierlich bestimmt werden kann. In der wissenschaftlichen Literatur werden auch zunehmend Verfahren publiziert, die mittels maschinellen Lernens oder anderer Methoden der künstlichen Intelligenz den Blutdruck aus einem einzelnen Sensor bestimmen wollen.

Alle diese "Cuffless"-Messverfahren haben zumindest zwei Nachteile. Diese Verfahren können den Absolutwert des Blutdruckes nicht bestimmen und brauchen somit eine Kalibration meist auf den mittels Manschette am Oberarm oder am Handgelenk gemessenen Blutdruck. Weiters verändern andere physiologischen Ereignisse sowohl die zeitlichen Differenzen als auch die Formen der Pulswellen, ohne dass eine Veränderung des Blutdruckes auftritt. Dadurch verändert sich das mathematische Modell, das der Berechnung des Blutdruckes zu Grunde liegt und die Ergebnisse werden verfälscht. Die Ursachen dafür sind Veränderungen des Gefäßwiderstandes durch die glatte Gefäßmuskulatur, die sich öffnen (Vasodilatation) und auch schließen (Vasokonstriktion) können. Dieses physiologische Phänomen wird ständig vom vegetativen Nervensystem gesteuert und führt dazu, dass das Kalibrationsintervall für diese Messverfahren recht kurz sein muss.

Die WO 2020 176206 A1 beschreibt ein System, bei dem mittels Armmanschette eine Kalibration durchgeführt werden kann. Diese Methode bzw. die dazu passenden Geräte benötigten jedoch zwei Sensoren, nämlich den Pulsationssensor sowie die Armmanschette.

Die US 2019 0059825 A1 beschreibt ein selbst-kalibrierendes System mit einer pneumatischen Fingermanschette. Im Wesentlichen wird eine sogenannte "oszillometrische" Messung am Finger mittels der luftgefüllten Blase (Manschette) intermittierend durchgeführt und diese Werte können dann zur Kalibrierung eines Systems - vorzugweise eines Systems das mit der "Pulse Decomposition Analyse" arbeitet - herangezogen werden.

Am Markt beginnt sich wegen der oben genannten Nachteile der "Cuffless"-Verfahren die sogenannte "Vascular Unloading Technique" durchzusetzen, die auf eine Publikation von Peňáz (Digest of the 10th International Conference on Medical and Biological Engineering 1973 Dresden) zurückgeht, bei der ein Finger durchleuchtet wird und durch eine Servoregelung der registrierte Fluss konstant gehalten wird.

Die Patentschrift EP 2 854 626 B1 beschreibt ein neuartiges Verfahren der sogenannten "Vascular Control Technique" inklusive des dazugehörigen Geräts, das einen nur sehr langsam veränderlichen Anpressdruck an die Extremität (meist Finger) aufbringt, um so dem mittleren arteriellen Blutdruck zu folgen. Die US 10 285 599 A1 beschreibt verschiedene Messmodi und ergänzende Elemente, die für eine Verwendung als tragbares Gerät wichtig sind.

Sowohl bei der "Vascular Unloading Technique" als auch bei der "Vascular Control Technique" wird während der Messung ständig ein Druck meist auf einen Finger ausgeübt. In der EP 1 179 991 B1 wird u.a. ein Doppelfingersensor beschrieben, bei dem zwei benachbarte Finger abwechselnd mit Druck beaufschlagt und gemessen werden können. In der EP 3 419 515 B1 wird ebenfalls ein Doppelfingersystem beschrieben, bei dem die beiden benachbarten Finger auf einem Körper zu liegen kommen, der einer Computermouse ähnelt. So kann jeweils an einem Finger die Messung durchgeführt werden, während der andere Finger rastet.

In der oben erwähnten US 10 285 599 wird ein Messmodus für die "Vascular Control Technique" beschrieben, bei der sich der Druck an einer Extremität (z.B. Finger) nach einer Messung auf rund 30-40 mmHg absenkt und nur mehr die Herzfrequenz misst. Der Finger kann so wiederum rasten, während auf die nächste Messung gewartet wird. Dies hat allerdings den Nachteil, dass während dieser sogenannten "Idle-Phase" keine vollständigen Herz-Kreislaufwerte vorhanden sind.

Aus dem Preprint von medRxiv mit dem Titel "A novel art of continuous non-invasive blood pressure measurement" (FORTIN et al.) ist ein am Finger tragbarer Sensor zur kontinuierlichen Messung des Blutdrucks (BP) und abgeleiteter kardiovaskulärer Variablen bekannt geworden. Es handelt sich um eine kompakte Messvorrichtung für die kontinuierliche, nicht-invasive arterielle Blutdrucküberwachung. Das gemessene pulsierende Blutdrucksignal enthält Informationen zur Ableitung der Herzleistung und anderer hämodynamischer Variablen. Weitere Messverfahren sind bekannt aus US2013/060152A1.

Ziel der Erfindung ist es eine Messvorrichtung und ein Verfahren zur kontinuierlichen, nicht-invasiven Bestimmung zumindest eines Herz-Kreislaufparameters, vorzugsweise des arteriellen Blutdrucks, an einer Extremität, derart weiter zu bilden, dass eine kontinuierliche Bestimmung der Parameter ermöglicht wird, wobei für die zu vermessende Extremität lange andauernde Druckbelastungen vermieden werden sollen. Weiters soll ein kompaktes, aus wenigen Einzelteilen bestehendes System verwirklicht werden, das auch in eine tragbare Einheit integrierbar ist.

Diese Aufgabe wird durch eine Messvorrichtung gemäß Anspruch 1 und ein Messverfahren gemäß Anspruch 5 erfüllt. Vorteilhafte Ausführungsvarianten werden in den abhängigen Ansprüchen offenbart.

Die gegenständliche Anmeldung beschreibt ein Messverfahren und eine Messvorrichtung, mit dem bzw. mit der ständig alle Herz-Kreislaufwerte eines Menschen bestimmt werden können, obwohl nur relativ kurz während eines Messvorganges ein Druck auf eine Extremität (z.B. Finger) ausgeübt wird.

Das erfindungsgemäße Verfahren weist grundsätzlich zwei unterschiedliche Betriebsmodi auf. Als erstes wird eine Messphase durchgeführt, bei der sich der Druck auf die Extremität im Sensor des Blutdruckmessgerätes verändern kann. Es wird ein Absolutwert bzw. die Absolutwerte des Blutdruckes gemessen und in weiterer Folge alle notwendigen Herz-Kreislaufparameter bestimmt.

Diese Herz-Kreislaufparameter sind zumindest der arterielle Blutdruck als kontinuierliches pulsatiles Signal p_{A}(t), sowie als systolischer (sBP), diastolischer (dBP) und mittlerer arterieller Blutdruck (mBP) für jeden Herzschlag. Optional können auch weitere kardiovaskuläre Werte (wie z.B. das Herzzeitvolumen (Cardiac Output CO), das Schlagvolumen (SV), der systemische Gefäßwiderstand (SVR), etc.), dynamische Variablen (wie z.B. Pulse Pressure Variation PPV bzw. Schlagvolumensvariation SVV) oder Parameter des vegetativen / autonomen Nervensystems (wie z.B. Barorezeptorreflexsensitivität BRS, Blutdruck- oder Herzratenvariabilität BPV / HRV, etc.) bestimmt werden.

Während der Messphase wird ein mathematisches Modell mit den gemessenen Herz-Kreislaufwerten gespeist bzw. kalibriert. Das mathematische Modell kann in verschiedensten Ausprägungen vorliegen. Einerseits kann das Modell aus experimentell ermittelten a-priori Wissen aufgebaut sein und die aus der Messphase gewonnen Werte parametrisieren das vorliegende Modell. Andererseits kann sich das Modell mittels maschineller Lernmethoden aus den gemessenen Werten selbst aufbauen. Alle hybriden Formen sind selbstverständlich ebenso möglich.

Nach der Messphase beginnt der zweite Teil der Methode: Ist das mathematische Modell hinreichend genau bestimmt, dann senkt sich der Anpressdruck im Blutdruckmessgerät auf einen minimalen Wert der ausreichend ist, um auch weiterhin die Pulsationen zu bestimmen, die durch den Volumenstrom in der Arterie entstehen. Durch das Absenken des Druckes verändert sich die Amplitude der Pulsationen, aber auch die Form der Pulsationen. Die Pulsationen werden dem mathematischen Modell zugeführt und das Modell schätzt bzw. interpoliert daraus neue Herz-Kreislaufparameter. So können diese Parameter bestimmt werden, ohne dass ein störender Druck auf die Extremität ausgeübt wird.

Das mathematische Modell ist auch in der Lage einen etwaigen Fehler zu den wahren, in einer Messphase bestimmten Herz-Kreislaufparameter zu bestimmen. Wird der Fehler zu groß, dann wird im Blutdruckmessgerät eine neue Messphase gestartet, bei der wiederum ein Druck im Sensor des Blutdruckmessgerätes auf die Extremität ausgeübt wird. Ebenso kann nach einer bestimmen Zeit eine neue Messphase gestartet werden. In einer neuen Messphase kann das mathematische Modell komplett neu aufgebaut werden. Es können aber auch Teiles des Modells aus der vergangenen Messphase wiederverwendet werden, um so z.B. die Zeit für das maschinelle Lernen und somit die Messphase zu verkürzen.

Ein weiterer Vorteil der vorliegenden Erfindung liegt darin, dass nur ein einzelner, im Idealfall tragbarer, Sensor verwendet werden muss.

Die Erfindung wird im Folgenden anhand von schematischen Darstellungen und Diagrammen näher erläutert:
- Fig. 1: eine erfindungsgemäße Messvorrichtung zur Bestimmung des arteriellen Blutdrucks in einer schematischen Darstellung;
- Fig. 2: eine Blockdarstellung eines erfindungsgemäßen Verfahrens zur Bestimmung des arteriellen Blutdrucks, wechselnd zwischen einer Messphase und einer Interpolationsphase;
- Fig. 3: eine Variante der Messvorrichtung gemäß Fig. 1, bei welcher der pulsatile Anteil des Drucks mittels Photoplethysmographie bestimmt wird;
- Fig. 4: eine Variante der Messvorrichtung gemäß Fig. 1, bei welcher aus einem oszillometrischen Signal der Absolutwert des Blutdruckes gewonnen wird; sowie
- Fig. 5: eine Blockdarstellung einer Variante des erfindungsgemäßen Verfahrens mit einer Initialphase.

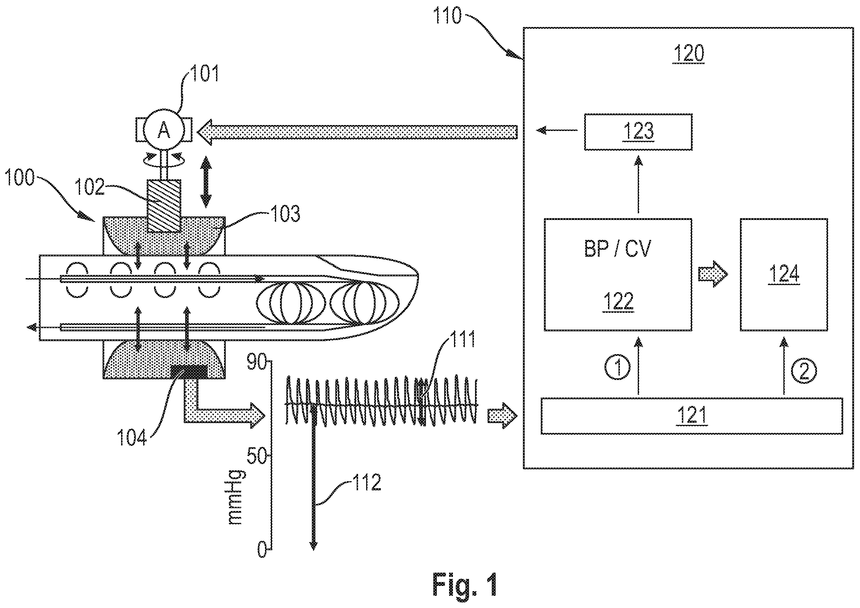

In Fig. 1 wird eine Ausführungsvariante der erfindungsgemäßen Messvorrichtung zur kontinuierlichen, nicht-invasiven Bestimmung zumindest eines Herz-Kreislaufparameters, beispielsweise ein Blutdruckmessgerätes dargestellt, das in diesem Fall mit einem Aufnahmeelement 100 (z.B. einem Finger-Cuff) auf dem Finger einer Hand angebracht ist. Die Messvorrichtung besteht im Wesentlichen aus einem Aktor 101, der über einen Stößel oder Kolben 102 einen Druck auf eine flexible Blase 103 ausübt, die im Aufnahmeelement 100 angeordnet ist. Vorzugsweise ist die Blase 103 mit einer Flüssigkeit oder einem Gas gefüllt, so dass der Druck in der Blase 103 auch auf den Finger und dann in weiterer Folge auf die Arterie in dem Finger wirken kann.

Der Druck in der Blase 103 wird mit einem Drucksensor 104 gemessen. In der vorliegenden Ausführungsvariante handelt es sich um einen hochauflösenden Drucksensor 104, der auch als Drucksensor für die arteriellen Pulse bzw. den pulsatilen Anteil des Drucksignals fungieren kann. Dazu muss der Drucksensor 104 eine hinreichende Auflösung haben und Druckänderungen von mindestens 0,01 mmHg (0,013 mbar) bei einer oberen Grenzfrequenz von mindestens 40 Hz erfassen können.

Diese vorliegende Methode funktioniert sehr gut, wenn die flexible Blase 103 vorzugsweise mit einem inkompressiblen Fluid, beispielsweise einer Flüssigkeit, gefüllt ist. Aber auch mit Gas (z.B. Luft) können die Pulsationen hinreichend übertragen werden. In Ausführungsvarianten mit einer luftgefüllten Blase 103 können anstelle eines einzelnen Kolbens 102 auch eine Luftpumpe sowie ein oder mehrere Ventile erforderlich sein (nicht dargestellt).

Der Drucksensor 104 misst somit den Absolutwert 112 des Druckes in der Blase 103 und auch die arteriellen Pulsationen, bzw. den pulsatilen Anteil 111 des Drucksignals. Der Absolutwert 112 des Druckes entspricht dabei in einem elektrischen Äquivalent dem Gleichanteil (DC), die arteriellen Pulsationen 111 entsprechen dem Wechselanteil (AC) des Drucksensorsignales. Das Signal wird nun der Steuereinheit 110 des Blutdruckmessgerätes zugeführt, in der vorliegenden Ausführungsvariante ist dies ein Microcontroller 120.

Der Microcontroller 120 beinhaltet zumindest die folgenden Elemente: Rechnereinheit bzw. Microcomputer, Speicher für den Programmcode, Arbeitsspeicher, Analog-Digitalwandler, Digital-Analogwandler, Bauelemente zur Spannungserzeugung und weitere mehr. Beispielsweise kann ein Microcontroller verwendet werden, der bereits die meisten Funktionen in einem Bauteil integriert zur Verfügung stellt. Der Regler kann aber auch mit anderen Methoden aufgebaut werden, wie z.B. anhand von analogen Schaltungen.

Im Microcontroller 120 sind folgende Elemente vorzugsweise in einem Softwarecode abgebildet: Signaldetektor 121, Messeinheit 122 für den Blutdruck BP und den weiteren Herz-Kreislaufparametern CV, eine Steuereinheit 123 für den Aktor 101 sowie ein mathematisches Modell 124. Zusätzlich können für die Bedienung des Gerätes Ein- und Ausgabeelemente vorgesehen sein, die hier nicht dargestellt sind.

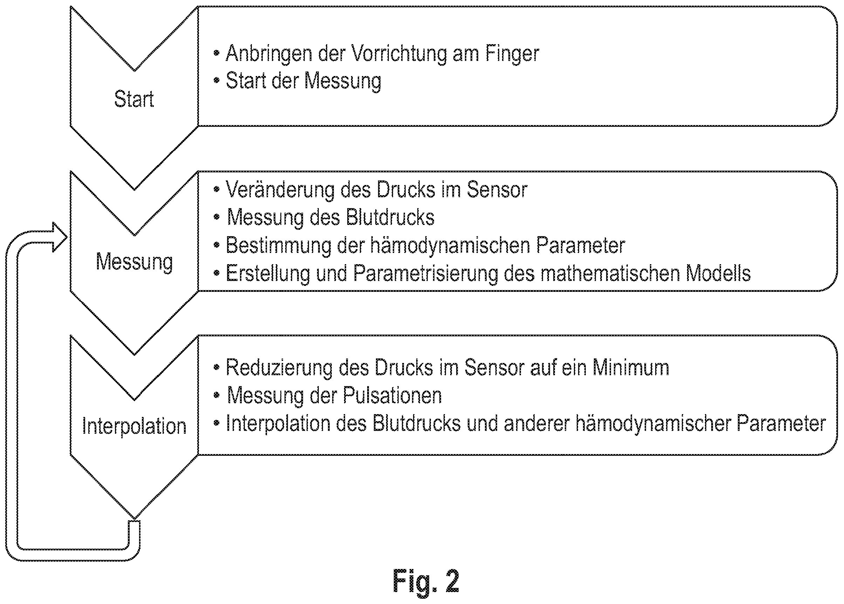

In der Fig. 2 ist eine einfache Ablauflogik des erfindungsgemäßen Messverfahrens dargestellt: In einer Startphase wird das Blutdruckmessgerät an die Extremität angebracht. Danach kann die Messung gestartet werden. In der Messphase ① wird der Druck in der flexiblen Blase 103 verändert, wobei sowohl der Absolutwert 112 des Druckes in der Blase 103 als auch die arteriellen Pulsationen 111 vom Drucksensor 104 gemessen werden.

Für die Bestimmung des Blutdruckes in der Arterie der Extremität können bekannte Verfahren wie die "Vascular Control Technique", die "Vascular Unloading Technique" oder auch das einfache, oszillometrische Verfahren verwendet werden. Aus dem Blutdruck können nun auch mit der Hilfe von bekannten Verfahren die weiteren vorhin genannten Herz-Kreislaufparameter bestimmt werden. Die Steuerung des Blutdruckmessverfahrens wird dabei vorzugsweise in der Messeinheit 122 für den Blutdruck und den weiteren Herz-Kreislaufparametern des Microcontrollers 120 in Form eines Softwarecodes abgebildet. Über eine Steuereinheit 123 für den Aktor 101 wird der Druck in der flexiblen Blase 103 und in weiterer Folge am Finger verändert.

Diese gemessenen Herz-Kreislaufparameter werden einem mathematischen Modell 124 zugeführt. Das mathematische Modell 124 kann in verschiedensten Ausprägungen vorliegen. Einerseits kann das Modell 124 aus experimentell ermittelten a-priori Werten aufgebaut sein und die aus der Messphase ① gewonnen Werte parametrisieren das vorliegende Modell. Andererseits kann sich das Modell 124 mittels maschineller Lernmethoden aus den gemessenen Werten selbst aufbauen. Alle hybriden Formen sind selbstverständlich ebenso möglich. Neben den gemessenen Herz-Kreislaufparametern kann auch das Drucksignal aus dem Drucksensor 104, insbesondere der Absolutdruck 112 sowie der pulsatile Anteil 111 des Drucksignals dem mathematischen Modell 124 zugeführt werden.

Nach der Messphase ① beginnt der zweite Teil des Verfahrens: die Interpolationsphase ②. Wenn das mathematische Modell 124 hinreichend genau bestimmt ist, dann senkt sich der Anpressdruck und somit der Absolutdruck 112 im Blutdruckmessgerät auf einen minimalen Wert. Vorzugsweise soll die Höhe des Anpressdruckes in der Interpolationsphase ② ausreichend hoch sein, damit auch weiterhin die Pulsationen 111 auftreten und bestimmt werden können, die durch den Volumenstrom in der Arterie entstehen. Im Idealfall geht der Anpressdruck in der Interpolationsphase ② gegen Null bzw. ist Null, so dass der Sensor den Patienten nicht stört.

Durch das Absenken des Absolutdrucks 112 verändert sich die Amplitude der Pulsationen, bzw. des pulsatilen Anteils 111 des Signals, aber auch die Form der Pulsationen 111, dennoch bleiben gewisse Eigenschaften wie Zeitabstände, Frequenzinhalte, Segmente und Abschnitte des Pulses etc. zumindest ähnlich. Die Pulsationen 111 werden dem mathematischen Modell 124 zugeführt und das Modell "schätzt" bzw. interpoliert daraus neue Herz-Kreislaufparameter. "Schätzen" (engl. "Estimates") deutet hier an, dass maschinelle Lernverfahren bzw. Methoden aus dem Bereich "Artificial Intelligence" verwendet werden können. So können diese Parameter bestimmt werden, ohne dass ein lang andauernder, störender Druck auf die Extremität ausgeübt werden muss.

Das mathematische Modell ist auch in der Lage einen etwaigen Fehler zu den wahren, in einer Messphase ① bestimmten Herz-Kreislaufparameter zu bestimmen. Wird der Fehler zu groß, dann wird im Blutdruckmessgerät eine neue Messphase ① gestartet, bei der wiederum ein Druck im Sensor des Blutdruckmessgerätes auf die Extremität ausgeübt wird. Ebenso kann nach einer bestimmen Zeit eine neue Messphase ① gestartet werden. In einer neuen Messphase ① kann das mathematische Modell komplett neu aufgebaut werden. Es können aber auch Teile des Modells aus der vergangenen Messphase ① wiederverwendet werden, um so z.B. die Zeit für das maschinelle Lernen und somit die Messphase ① zu verkürzen.

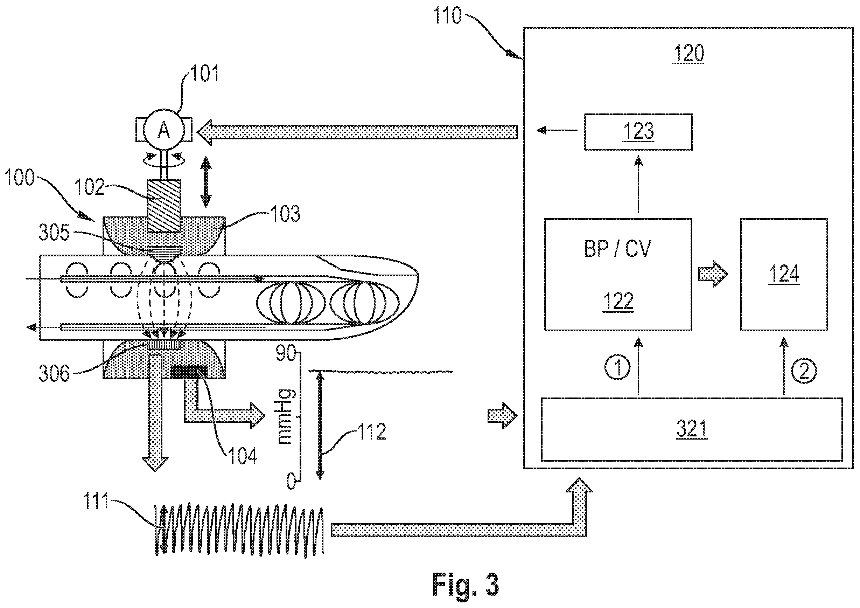

In der Fig. 3 wird eine weitere Ausführungsvariante der erfindungsgemäßen Messvorrichtung gezeigt. Es unterscheidet sich im Wesentlichen in der Messung der Pulsationen, bzw. des pulsatilen Anteils 111, die in dieser Variante mit der Hilfe von Lichtsensoren gemessen werden. Dort wo die flexible Blase 103 am Finger anliegt sind diese Lichtsensoren angebracht und sie bestehen auf zumindest einer Lichtquelle 305 und aus zumindest einem Lichtdetektor 306. Die Lichtquelle 305 ist vorzugsweise eine LED mit infrarotem Licht und strahlt durch den Finger. Das infrarote Licht wird durch die Erythrozyten in der Arterie absorbiert und abhängig von der Menge an Erythrozyten entsteht ein moduliertes Licht, das auf der anderen Seite des Fingers wieder austritt. Der Lichtdetektor 306 ist vorzugsweise eine Fotodiode und misst das modulierte Licht, das durch den Finger strahlt. Dieses Licht ist somit ein Maß für das Blutvolumen in der Arterie. Dieses Lichtsignal, das die Pulsationen 311 repräsentiert, wird dem Signaldetektor 121 des Microcontrollers 120 zugeführt.

Dennoch muss auch hier - wie bei der Ausführungsvariante gemäß Fig. 1 ein Drucksensor 104 vorhanden sein, der jedoch nur den Absolutwert 112 des Druckes in der flexiblen Blase 103 messen und dem Microcontroller 120 zuführen muss.

Die Verwendung von Lichtsensoren 305 und 306 hat den Vorteil, dass der Anpressdruck während der Interpolationsphase ② noch weiter Richtung Null gesenkt werden kann, weil ja theoretisch die Pulsationen durch die Volumensveränderungen der vom Anpressdruck unbeeinflussten Arterie durch das Licht entstehen können. Ohne Anpressdruck ist es hingegen schwierig, dass die Lichtsensoren 305 und 306 das Licht über die Haut ein- und auskoppeln. Auch bei den vorhin genannten sogenannten "Cuffless" oder auch "Ubiquitous" Messverfahren, die zumeist mit Lichtsensoren arbeiten, ist ein Anpressdruck vorhanden. Diese Sensoren werden oft mittels Armbandes (z.B. Fitnessuhr), Feder oder Klettverschluss am Körper befestigt, um die Ein- und Auskoppelung des Lichtes zu gewährleisten.

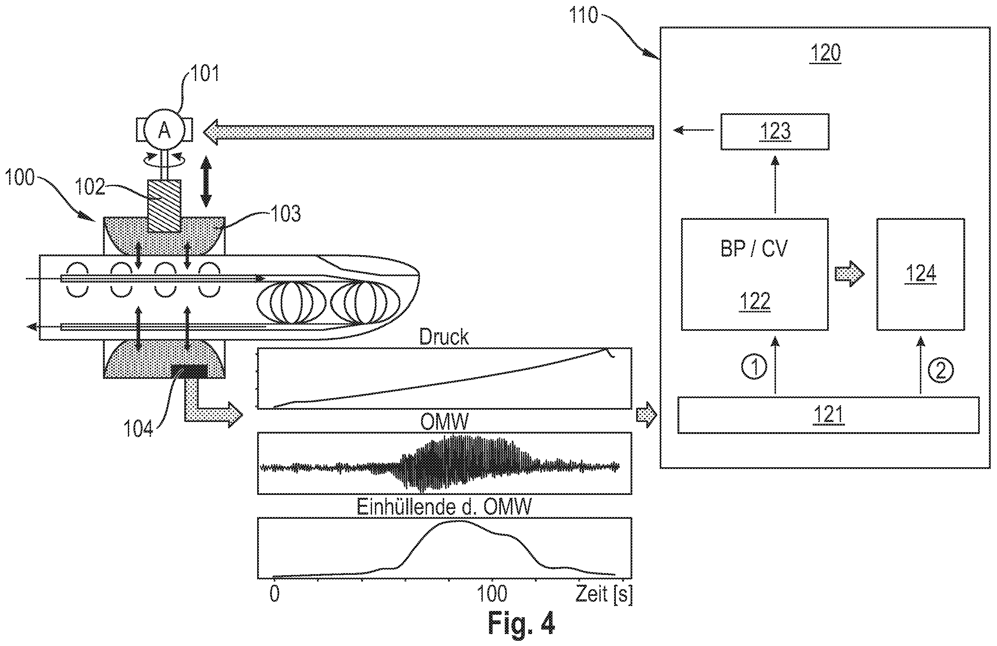

In Fig. 4 wird wiederum die Ausführungsvariante aus Fig. 1 dargestellt. Hier wird der Blutdruck allerdings nicht kontinuierlich gemessen. Die Messeinheit 122 für den Blutdruck BP und weiterer Herz-Kreislaufparametern CV legt beispielsweise eine Druckrampe an den Finger an (siehe Teilbild "Druck") und ein oszillometrisches Signal "OMW" bzw. die "Einhüllende des OMW" (siehe mittleres bzw. unteres Teilbild) wird bestimmt. Daraus können in bekannter Weise der systolische, diastolische sowie der mittlere arterielle Blutdruck bestimmt werden. Grundsätzlich könnte bereits mit dieser Methode das mathematische Modell 124 gespeist werden, um in der folgenden Interpolationsphase ② zumindest den Blutdruck zu bestimmen. Diese einfache Variante ist auch Gegenstand der vorliegenden Anmeldung.

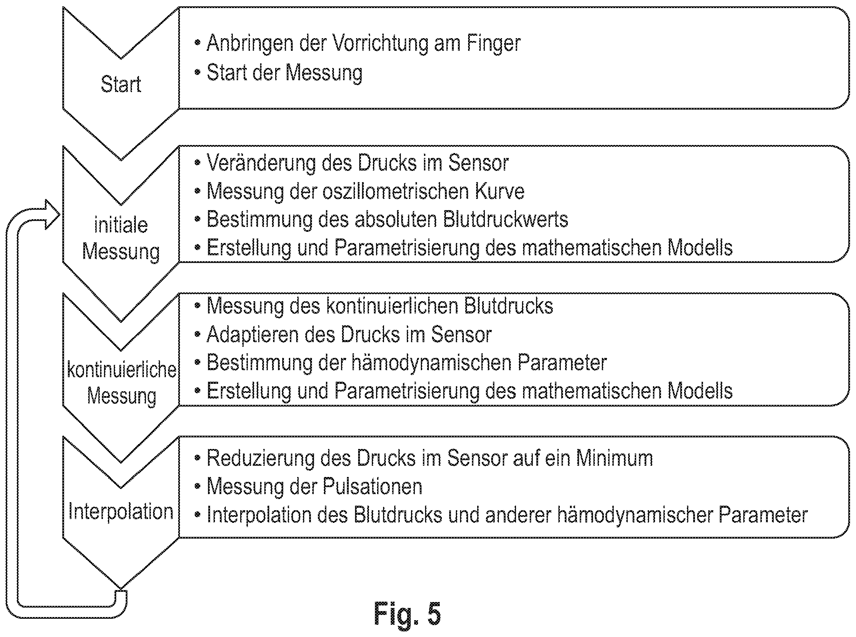

Dieses oszillometrische Verfahren kann auch initial am Beginn einer Messphase ① durchgeführt werden, wie im Ablaufdiagramm in Fig. 5 dargestellt ist. Mit den Werten aus dieser initialen Messphase kann eine zweite kontinuierliche Messphase angesteuert und kalibriert werden, um die Herz-Kreislaufwerte noch genauer messen zu können. Hier wird das mathematische Modell 124 sowohl in der initialen Messphase als auch in der zweiten kontinuierlichen Messphase erstellt und parametrisiert, damit mit diesem Modell 124 in der Interpolationsphase ② die Herz-Kreislaufwerte bestimmt werden können, ohne dass ein Druck auf die Extremität ausgeübt wird.

## Patentansprüche

1. Messvorrichtung zur kontinuierlichen, nicht-invasiven Bestimmung zumindest eines Herz-Kreislaufparameters, vorzugsweise des arteriellen Blutdrucks, an einer Extremität, die eine Arterie enthält,
mit einem Aufnahmeelement (100) das an der Extremität anbringbar ist und eingerichtet ist die Extremität zumindest teilweise zu umfassen,
mit einer sich am Aufnahmeelement (100) abstützenden, auf die Extremität wirkenden, flexiblen Blase (103), die mit einem Fluid gefüllt ist,
wobei im oder am Aufnahmeelement (100) ein Aktor (101) angeordnet ist, der geeignet ist, den Druck in der flexiblen Blase (103) zu variieren,
dieflexible Blase (103) einen Drucksensor (104) in Kontakt mit dem Fluid in der flexiblen Blase (103) aufweist, wobei der Drucksensor (104) geeignet ist, den Absolutwert (112) des Drucks kontinuierlich zu messen,
die Messvorrichtung eine Einrichtung aufweist, die geeignet ist die Pulsationen (111) zu messen, die durch den Volumenstrom in der Arterie entstehen,
die Einrichtung zur Messung der Pulsationen (111), die durch den Volumenstrom in der Arterie entstehen, ein photoplethysmographisches System mit zumindest einer Lichtquelle (305) und zumindest einem Lichtdetektor (306) aufweist, sowie
die Messvorrichtung eine Steuereinheit (110) aufweist, die zumindest folgende Elemente beinhaltet:
- eine Signaldetektionseinheit (121), die eingerichtet ist, den Absolutwert (112) des Druckes sowie die Pulsationen (111), die durch den Volumenstrom in der Arterie entstehen, aufzuzeichnen,
- eine Messeinheit (122) zur Bestimmung des zumindest einen Herz-Kreislaufparameters,
- eine Steuereinheit (123) für den Aktor (101), die eingerichtet ist, den Druck in der flexiblen Blase (103) zu variieren, sowie
- ein mathematisches Modell (124), eingerichtet für die Interpolation des zumindest einen Herz-Kreislaufparameters basierend auf den Pulsationen (111), die durch den Volumenstrom in der Arterie entstehen, gemessen von dem photoplethysmographischen System bei einem auf ein Minimum abgesenkten Druck in der flexiblen Blase (103) während einer Interpolationsphase der Messvorrichtung.

2. Messvorrichtung nach Anspruch 1, **dadurch gekennzeichnet, dass** die Steuereinheit (110) zumindest zwei verschiedene Betriebsmodi aufweist: eine Messphase und eine Interpolationsphase.

3. Messvorrichtung nach Anspruch 1 oder 2, **dadurch gekennzeichnet, dass** der Drucksensor (104) zur Messung des Absolutwerts (112) des Drucks eingerichtet ist, auch die Pulsationen (111), die durch den Volumenstrom in der Arterie entstehen, zu messen.

4. Messvorrichtung nach einem der Ansprüche 1 bis 3, **dadurch gekennzeichnet, dass** die einzelnen Komponenten der Messvorrichtung in einer am Körper, vorzugsweise am Finger einer Hand, tragbaren Einheit integriert sind.

5. Verfahren zur kontinuierlichen, nicht-invasiven Bestimmung zumindest eines Herz-Kreislaufparameters, vorzugsweise des arteriellen Blutdrucks, an einer Extremität, die eine Arterie enthält, wobei die Extremität von einer flexiblen, mit einem Fluid gefüllten Blase (103) zumindest teilweise umfasst wird, und wobei ein Drucksensor (104) in der Blase (103) angeordnet ist, der ein Drucksignal p_{c}(t) generiert,
wobei der Druck in der flexiblen Blase (103) mit Hilfe eines auf das Fluid oder die flexible Blase (103) wirkenden Aktors verändert wird,
in einer Messphase
- der Absolutwert (112) des Drucks in der flexiblen Blase (103) variiert und gemessen wird,
- die Pulsationen (111) gemessen werden, die durch den Volumenstrom in der Arterie während der Messphase generiert werden,
- aus dem Absolutwert (112) und den Pulsationen (111) der zumindest eine Herz-Kreislaufparameter bestimmt wird,
- der zumindest eine Herz-Kreislaufparameter einem mathematischen Modell (124) zugeführt wird,
sowie danach in einer Interpolationsphase
- der Druck in der flexiblen Blase (103) auf ein Minimum reduziert wird,
- die Pulsationen (111) mit Hilfe eines photoplethysmographischen Systems mit zumindest einer Lichtquelle (305) und zumindest einem Lichtdetektor (306) gemessen werden, die durch den Volumenstrom in der Arterie während der Interpolationsphase generiert werden,
- die Pulsationen (111), die durch den Volumenstrom in der Arterie entstehen, dem mathematischen Modell (124) zugeführt werden, und
- aus dem mathematischen Modell (124) und den Pulsationen (111), die durch den Volumenstrom in der Arterie entstehen, der zumindest eine Herz-Kreislaufparameter interpoliert wird.

6. Verfahren nach Anspruch 5, **dadurch gekennzeichnet, dass** das mathematische Modell (124) eingerichtet ist Abweichungen und Fehler der Interpolation des zumindest einen Herz-Kreislaufparameters zu berechnen.

7. Verfahren nach Anspruch 6, **dadurch gekennzeichnet, dass** in Abhängigkeit des berechneten Fehlers des zumindest einen Herz-Kreislaufparameters ein Neustart der Messphase eingeleitet wird.

8. Verfahren nach Anspruch 5, **dadurch gekennzeichnet, dass** nach Ablauf einer vorgebbaren Zeitspanne ein Neustart der Messphase eingeleitet wird.

9. Verfahren nach einem der Ansprüche 5 bis 8, **dadurch gekennzeichnet, dass** während der Messphase die Pulsationen (111), die durch den Volumenstrom in der Arterie entstehen, dem mathematischen Modell (124) zugeführt werden.

10. Verfahren nach einem der Ansprüche 5 bis 9, **dadurch gekennzeichnet, dass** während der Messphase der Absolutwert (112) des Drucks dem mathematischen Modell (124) zugeführt wird.

11. Verfahren nach einem der Ansprüche 5 bis 9, **dadurch gekennzeichnet, dass** zur Bestimmung des zumindest einen Herz-Kreislaufparameters die Vascular Control Technique (VCT) angewandt wird.

12. Verfahren nach einem der Ansprüche 5 bis 9, **dadurch gekennzeichnet, dass** zur Bestimmung des zumindest einen Herz-Kreislaufparameters die Vascular Unloading Technique angewandt wird.

13. Verfahren nach einem der Ansprüche 5 bis 9, **dadurch gekennzeichnet, dass** zur Bestimmung des zumindest einen Herz-Kreislaufparameters ein oszillometrisches Verfahren angewandt wird.

14. Verfahren nach einem der Ansprüche 5 bis 13, **dadurch gekennzeichnet, dass** zur Bestimmung des zumindest einen Herz-Kreislaufparameters zuerst das oszillometrische Verfahren und danach die Vascular Control Technique oder die Vascular Unloading Technique durchgeführt wird.

## Claims

1. A measuring device for continuously, non-invasively determining at least one cardiovascular parameter, preferably arterial blood pressure, on an extremity containing an artery,
comprising a recording element (100), which can be attached to the extremity and is configured for at least partially enclosing the extremity, and
comprising a flexible, fluid-filled bladder (103), which is supported on the recording element (100) and acts on the extremity,
wherein
an actuator (101) suitable for varying the pressure in the flexible bladder (103) is arranged in or on the recording element (100),
the flexible bladder (103) has a pressure sensor (104) in contact with the fluid in the flexible bladder (103), the pressure sensor (104) being suitable for continuously measuring the absolute value (112) of the pressure,
the measuring device has a means suitable for measuring the pulsations (111) that occur as a result of the volume flow in the artery, and
the measuring device has a control unit (110), which includes at least the following elements:
- a signal detection unit (121), which is configured for recording the absolute value (112) of the pressure as well as the pulsations (111) that occur as a result of the volume flow in the artery,
- a measuring unit (122) for determining the at least one cardiovascular parameter,
- a control unit (123) for the actuator (101), which is configured for varying the pressure in the flexible bladder (103), and
- a mathematical model (124) configured for interpolating the at least one cardiovascular parameter, on the basis of the pulsations (111) that occur as a result of the volume flow in the artery, while a pressure in the flexible bladder (103) is reduced to a minimum during an interpolation phase of the measuring device.

2. The measuring device according to claim 1, **characterised in that** the control unit (110) has at least two different operating modes: a measurement phase and an interpolation phase.

3. The measuring device according to claim 1 or 2, **characterised in that** the pressure sensor (104) for measuring the absolute value (112) of the pressure is also configured for measuring the pulsations (111) that occur as a result of the volume flow in the artery.

4. The measuring device according to any one of claims 1 to 3, **characterised in that** the individual components of the measuring device are integrated in a unit that can be worn on the body, preferably on the finger of one hand.

5. A method for continuously, non-invasively determining at least one cardiovascular parameter, preferably arterial blood pressure, on an extremity containing an artery, wherein the extremity is at least partially enclosed by a flexible, fluid-filled bladder (103), and wherein a pressure sensor (104), which generates a pressure signal p_{c}(t), is arranged in the bladder (103),
wherein
the pressure in the flexible bladder (103) is varied by means of an actuator acting on the fluid or the flexible bladder (103),
in a measurement phase,
- the absolute value (112) of the pressure in the flexible bladder (103) is varied and measured,
- the pulsations (111) generated by the volume flow in the artery during the measurement phase are measured,
- the at least one cardiovascular parameter is determined from the absolute value (112) and the pulsations (111),
- the at least one cardiovascular parameter is fed to a mathematical model (124),
and subsequently, in an interpolation phase,
- the pressure in the flexible bladder (103) is reduced to a minimum,
- the pulsations (111) generated by the volume flow in the artery during the interpolation phase are measured using a photoplethysmographic system comprising at least one light source (305) and at least one light detector (306),
- the pulsations (111) that occur as a result of the volume flow in the artery are fed to the mathematical model (124), and
- the at least one cardiovascular parameter is interpolated from the mathematical model (124) and the pulsations (111) that occur as a result of the volume flow in the artery.

6. The method according to claim 5, **characterised in that** the mathematical model (124) is configured for calculating deviations and errors of the interpolation of the at least one cardiovascular parameter.

7. The method according to claim 6, **characterised in that** a restart of the measurement phase is initiated as a function of the calculated error of the at least one cardiovascular parameter.

8. The method according to claim 5, **characterised in that** a restart of the measurement phase is initiated after a specifiable period of time has elapsed.

9. The method according to any one of claims 5 to 8, **characterised in that** the pulsations (111) that occur as a result of the volume flow in the artery are fed to the mathematical model (124) during the measurement phase.

10. The method according to any one of claims 5 to 9, **characterised in that** the absolute value (112) of the pressure is fed to the mathematical model (124) during the measurement phase.

11. The method according to any one of claims 5 to 9, **characterised in that** the vascular control technique (VCT) is used to determine the at least one cardiovascular parameter.

12. The method according to any one of claims 5 to 9, **characterised in that** the vascular unloading technique is used to determine the at least one cardiovascular parameter.

13. The method according to any one of claims 5 to 9, **characterised in that** an oscillometric method is used to determine the at least one cardiovascular parameter.

14. The method according to any one of claims 5 to 13, **characterised in that** first the oscillometric method and then the vascular control technique or the vascular unloading technique is carried out to determine the at least one cardiovascular parameter.

## Revendications

1. Dispositif de mesure pour la détermination non invasive continue d'au moins un paramètre cardiovasculaire, de préférence de la pression sanguine artérielle, au niveau d'une extrémité qui contient une artère,
avec un élément de réception (100) qui peut être fixé au niveau de l'extrémité et est conçu pour comprendre au moins en partie l'extrémité, avec une vessie souple (103) reposant sur l'élément de réception (100), agissant sur l'extrémité, laquelle vessie souple est remplie d'un fluide, dans lequel
un actionneur (101) est disposé dans ou au niveau de l'élément de réception (100), lequel actionneur est approprié pour faire varier la pression dans la vessie souple (103),
la vessie souple (103) comporte un capteur de pression (104) en contact avec le fluide dans la vessie souple (103), le capteur de pression (104) étant approprié pour mesurer en continu la valeur absolue (112) de la pression, le dispositif de mesure comportant un moyen qui est approprié pour mesurer les pulsations (111) qui résultent du débit volumétrique dans l'artère,
le moyen pour la mesure des pulsations (111) qui résultent du débit volumétrique dans l'artère comporte un système photopléthysmographique avec au moins une source de lumière (305) et au moins un détecteur de lumière (306), et
le dispositif de mesure comportant une unité de commande (110), laquelle inclut au moins les éléments suivants :
- une unité de détection de signaux (121), laquelle est conçue pour enregistrer la valeur absolue (112) de la pression ainsi que les pulsations (111) qui résultent du débit volumétrique dans l'artère,
- une unité de mesure (122) pour la détermination de l'au moins un paramètre cardiovasculaire,
- une unité de commande (123) pour l'actionneur (101), laquelle est conçue pour faire varier la pression dans la vessie souple (103), et
- un modèle mathématique (124), conçu pour l'interpolation de l'au moins un paramètre cardiovasculaire sur la base des pulsations (111) qui résultent du débit volumétrique dans l'artère, mesurées par le système photopléthysmographique lorsqu'une pression dans la vessie souple (103) est abaissée à un minimum pendant une phase d'interpolation du dispositif de mesure.

2. Dispositif de mesure selon la revendication 1, **caractérisé en ce que** l'unité de commande (110) comporte au moins deux modes de fonctionnement différents : une phase de mesure et une phase d'interpolation.

3. Dispositif de mesure selon la revendication 1 ou 2, **caractérisé en ce que** le capteur de pression (104) pour la mesure de la valeur absolue (112) de la pression est conçu pour mesurer également les pulsations (111) qui résultent du débit volumétrique dans l'artère.

4. Dispositif de mesure selon l'une des revendications 1 à 3, **caractérisé en ce que** les composants individuels du dispositif de mesure sont intégrés dans une unité portable au niveau du corps, de préférence au niveau du doigt d'une main.

5. Procédé pour la détermination non invasive continue d'au moins un paramètre cardiovasculaire, de préférence de la pression sanguine artérielle, au niveau d'une extrémité qui contient une artère, dans lequel l'extrémité est comprise au moins en partie dans une vessie souple (103) remplie d'un fluide, et dans lequel un capteur de pression (104) est disposé dans la vessie (103), lequel capteur de pression génère un signal de pression p_{c}(t),
dans lequel
la pression dans la vessie souple (103) est modifiée à l'aide d'un actionneur agissant sur le fluide ou la vessie souple (103),
dans une phase de mesure
- la valeur absolue (112) de la pression dans la vessie souple (103) est amenée à varier et mesurée,
- les pulsations (111) sont mesurées, lesquelles pulsations sont générées par le débit volumétrique dans l'artère pendant la phase de mesure,
- l'au moins un paramètre cardiovasculaire est déterminé à partir de la valeur absolue (112) et des pulsations (111),
- l'au moins un paramètre cardiovasculaire est introduit dans un modèle mathématique (124),
et ensuite, dans une phase d'interpolation
- la pression dans la vessie souple (103) est réduite à un minimum,
- les pulsations (111) sont mesurées à l'aide d'un système photopléthysmographique avec au moins une source de lumière (305) et au moins un détecteur de lumière (306), lesquelles pulsations sont générées par le débit volumétrique dans l'artère pendant la phase d'interpolation,
- les pulsations (111) qui résultent du débit volumétrique dans l'artère sont introduites dans le modèle mathématique (124), et
- l'au moins un paramètre cardiovasculaire est interpolé à partir du modèle mathématique (124) et des pulsations (111) qui résultent du débit volumétrique dans l'artère.

6. Procédé selon la revendication 5, **caractérisé en ce que** le modèle mathématique (124) est conçu pour calculer des déviations et des erreurs de l'interpolation de l'au moins un paramètre cardiovasculaire.

7. Procédé selon la revendication 6, **caractérisé en ce que**, en fonction de l'erreur calculée de l'au moins un paramètre cardiovasculaire, un redémarrage de la phase de mesure est lancé.

8. Procédé selon la revendication 5, **caractérisé en ce que**, après écoulement d'une durée pouvant être prédéfinie, un redémarrage de la phase de mesure est lancé.

9. Procédé selon l'une des revendications 5 à 8, **caractérisé en ce que**, pendant la phase de mesure, les pulsations (111) qui résultent du débit volumétrique dans l'artère sont introduites dans le modèle mathématique (124).

10. Procédé selon l'une des revendications 5 à 9, **caractérisé en ce que**, pendant la phase de mesure, la valeur absolue (112) de la pression est introduite dans le modèle mathématique (124).

11. Procédé selon l'une des revendications 5 à 9, **caractérisé en ce que**, pour la détermination de l'au moins un paramètre cardiovasculaire, la technique dite « vascular control » (*Vascular Control Technique,* VCT) est appliquée.

12. Procédé selon l'une des revendications 5 à 9, **caractérisé en ce que**, pour la détermination de l'au moins un paramètre cardiovasculaire, la technique dite « vascular unloading » (*Vascular Unloading Technique*) est appliquée.

13. Procédé selon l'une des revendications 5 à 9, **caractérisé en ce que**, pour la détermination de l'au moins un paramètre cardiovasculaire, un procédé oscillométrique est appliqué.

14. Procédé selon l'une des revendications 5 à 13, **caractérisé en ce que**, pour la détermination de l'au moins un paramètre cardiovasculaire, le procédé oscillométrique est tout d'abord mis en œuvre, et la technique dite « vascular control » ou la technique dite « vascular unloading » est ensuite mise en œuvre.
